# EUROPEAN PATENT APPLICATION

(11) **EP 1 533 041 A1**
(43) Date of publication of application: **25.05.2005**
(21) Application number: 03717695.5
(22) Date of filing: 22.04.2003
(51) Int. Cl.: B05B 9/08, B05B 17/04, B05B 15/00, A61M 11/00, A61M 11/08, A61L 2/22, C02F 1/46

(54) **ELECTROLYZED WATER SPRAYING DEVICE**

(30) Priority: 08.05.2002 JP 2002132596
(71) Applicant: MIKUNI CORPORATION, Chiyoda-ku, Tokyo 101-0021 (JP)
(72) Inventor: KASUYA, Shoji, MIKUNI CORP. Odawara Branch, Odawara-shi, Kanagawa 250-0055 (JP); HASHIMOTO, Hiroshi, MIKUNI CORP. Odawara Branch, Odawara-shi, Kanagawa 250-0055 (JP)
(74) Representative: Betten & Resch
(86) International application number: PCT/JP2003/005094
(87) International publication number: WO 2003/095102

(57) **Abstract**

An electrolytic aqueous solution tank (4) and a waste liquid tank (32) are disposed above a pump (6) to prevent electrolytic water from flowing to the waste liquid tank from the electrolytic aqueous solution tank under a siphoning effect. The electrolytic aqueous solution tank (4) and the waste liquid tank (32) are formed separately from the housing (2) so that both tanks can be attached to and detached from the housing (2). The electrolytic aqueous solution tank (4) and the waste liquid tank (32) are urged against the housing (2) by means of an elastic member (66) attached to the tank (4) and an elastic member (70) attached to the tank (32). As a result, when the waste liquid tank (32) is removed, it is inclined and hence there is no fear of spilling of waste liquid.

## Description

### FIELD OF ART

The present invention relates to a portable, electrolytic water atomizer for electrolyzing an electrolytic aqueous solution to produce anode electrolytic water and cathode electrolytic water both having various functions and atomizing the thus-produced water.

### BACKGROUND ART

A technique has already been well known in which an electrolytic dilute aqueous solution of, for example, NaCl is electrolyzed by using an electrolytic bath having an inert electrode formed of platinum or platinum alloy in the interior thereof, through or without through a charged film of an ion exchange resin or an uncharged film having a microporous structure as diaphragm, and anode electrolytic water (acidic water) of a low pH value produced on the anode side is withdrawn and utilized for sterilization and disinfection.

Since hypochlorous acid is produced within the anode electrolytic water produced on the anode side, the strong oxidizing action and chlorinating action of the hypochlorous acid are utilized for sterilization and disinfection. Such a utilization mode is spread in medical institutions, etc. Besides, since ozone and dissolved oxygen contained within the anode electrolytic water in a trace amount exhibit a granulation generation accelerating operation, their use as an aid to surgical treatment is also under study.

On the other hand, the cathode electrolytic water (alkaline water) produced on the cathode side is obtained by using tap water instead of the dilute electrolytic solution and electrolyzing the tap water, and has heretofore been used as drinking water.

In Japanese Unexamined Patent Publication No. Hei 8(1996)-243562, there is described an electrolytic water producing apparatus wherein a flow detector is disposed in a flow path of electrolytic water to detect an amount of water supplied to an electrolytic bath and an amount of electrolytic water produced, and the pH of the electrolytic water is kept constant. In many cases, such a conventional electrolytic water producing apparatus is relatively large-sized and is fixed to a place where the electrolytic water is used.

On the other hand, portable electrolytic water producing apparatus have also been known heretofore (PCT/JP95/01503, etc.).

The present inventors have so far developed portable electrolytic water producing apparatus (Japanese Unexamined Patent Publication Nos. 2002-52069 and 2002-65819). These electrolytic water producing apparatus are portable, small-sized and light-weight apparatus containing a battery and are constructed so that electrolytic water produced by the electrolysis of an electrolytic aqueous solution is sprayed as fine mist and is applied, for example to the skin. The apparatus are very convenient for use.

Fig. 12 shows an electrolytic water atomizer disclosed in the above Patent Publication No. 2002-52069. In the same figure, the numeral 500 denotes an electrolytic water atomizer, and electrolytic aqueous solution 504 in an electrolytic aqueous solution tank 502 is fed to an electrolytic bath 508 by means of a pump 506 and is electrolyzed therein. Anode electrolytic water (acidic water) produced on the anode side in the electrolytic bath 508 is sprayed from an atomizing unit 510 to the exterior and is applied, for example, to the skin.

On the other hand, cathode electrolytic water (alkaline water) produced on the cathode side of the electrolytic bath 508 passes through a discharge pipe 512 and is fed to a waste liquid tank 514, in which it is stored temporarily as waste liquid 516.

In the electrolytic water atomizer 500, since the electrolytic aqueous water tank 502 is positioned above the waste liquid tank 514, the discharge pipe 512 may function as a siphon and cause the electrolytic aqueous solution 504 in the upper electrolytic aqueous solution tank 502 to flow down into the waste liquid tank 514. So for preventing such an inconvenience, a special mechanism is provided. However, the provision of such a special mechanism gives rise to the problem that the number of part items and assembling man-hours increase. Further, for carrying the atomizer at all times, it is desirable that the size and weight be reduced.

### DISCLOSURE OF THE INVENTION

The present invention has been accomplished for solving the above-mentioned problem and it is an object of the invention to provide an electrolytic water atomizer capable of preventing an electrolytic aqueous solution from flowing down due to the foregoing siphoning effect without requiring provision of any special mechanism and attaining a further reduction in size and weight to such an extent as is suitable for carrying the atomizer.

It is another object of the present invention to provide an electrolytic water atomizer capable of preventing a spill of waste liquid from a waste liquid tank at the time of withdrawing the waste liquid from the interior of the waste liquid tank.

For achieving the above-mentioned objects the present invention resides in the following:
[1] An electrolytic water atomizer comprising a housing, an electrolytic aqueous solution tank provided within the housing, a pump for supplying an electrolytic aqueous solution stored in the electrolytic aqueous solution tank to the inlet side of an electrolytic bath, the electrolytic bath which is a flow type and is provided with an anode and a cathode opposed to each other in the interior thereof, an atomizing unit for spraying electrolytic water produced on one electrode side in the electrolytic bath to the exterior of the housing, a waste liquid tank, a discharge pipe which connects the electrolytic bath with the waste liquid tank to conduct electrolytic water produced on the other electrode side in the electrolytic bath to the waste liquid tank, a control unit for controlling operation of the atomizing unit, the electrolytic bath, and the pump, and a power supply for driving these components, wherein the electrolytic aqueous water tank and the waste liquid tank are disposed above the pump.
[2] An electrolytic water atomizer according to [1], wherein the waste liquid tank is formed separately from the housing so that it can be attached to and detached from the housing, an elastic member is attached to one of the waste liquid tank and the housing, the other of the waste liquid tank and the housing has a wall surface adapted to contact with and compress the elastic member, a first engaging means is provided in the waste liquid tank, a second engaging means for engagement with the first engaging means is provided in the housing, and the first engaging means and the second engaging means are kept engaged with each other with a repulsive force of the elastic member compressed by the wall surface.
[3] An electrolytic water atomizer according to [2], wherein the elastic member is an O-ring attached to a lower portion of the waste liquid tank, and the wall surface is an annular tapered wall surface formed in the housing.
[4] An electrolytic water atomizer according to [2], wherein a lid is provided in the housing, a first retaining means is provided in the lid, and a second retaining means for engagement with the first retaining means is provided in the waste liquid tank.
[5] An electrolytic water atomizer according to [2], wherein the electrolytic aqueous solution tank is formed separately from the housing so that it can be attached to and detached from the housing, an elastic member is attached to one of the electrolytic aqueous solution tank and the housing, the other of the electrolytic aqueous solution tank and the housing has a wall surface adapted to contact with and compress the elastic member, a first engaging means is provided in the electrolytic aqueous solution tank, a second engaging means for engagement with the first engaging means is provided in the housing, and the first engaging means and the second engaging means are kept engaged with each other with a repulsive force of the elastic member compressed by the wall surface.
[6] A electrolytic water atomizer according to [5], wherein the elastic member is an O-ring attached to a lower portion of the electrolytic aqueous solution tank, and the wall surface is an annular tapered wall surface formed in the housing.
[7] An electrolytic water atomizer according to [5], wherein a lid is provided in the housing, a first retaining means is provided in the lid, and a second retaining means for engagement with the first retaining means is provided in the waste liquid tank.

### BRIEF DESCRIPTION OF THE DRAWINGS

Fig. 1 is a schematic configuration diagram showing an example of an electrolytic water atomizer according to the present invention;
Fig. 2 is an explanatory diagram showing a state of spray of the electrolytic water atomizer of Fig. 1;
Fig. 3 is an enlarged configuration diagram showing an electrolytic bath used in the electrolytic water atomizer of Fig. 1;
Fig. 4 is a schematic configuration diagram showing another example of an electrolytic water atomizer according to the present invention;
Fig. 5 is an exploded perspective view showing an exploded state of main constituent parts of a further example of an electrolytic water atomizer according to the present invention;
Fig. 6 is a perspective view showing an assembled state of the main constituent parts of Fig. 5;
Fig. 7 is a sectional view of an electrolytic aqueous solution tank and a waste liquid tank both used in the electrolytic water atomizer of Fig. 5;
Fig. 8 is a sectional view of the waste liquid tank of Fig. 7 before mounting to a housing;
Fig. 9 is a sectional view showing a mounted state of the waste liquid tank of Fig. 8 to the housing;
Fig. 10 is a sectional view showing another example for a mounted state of a waste liquid tank to the housing;
Fig. 11 is a sectional view showing another example for a mounted state of an electrolytic aqueous solution tank to the housing; and

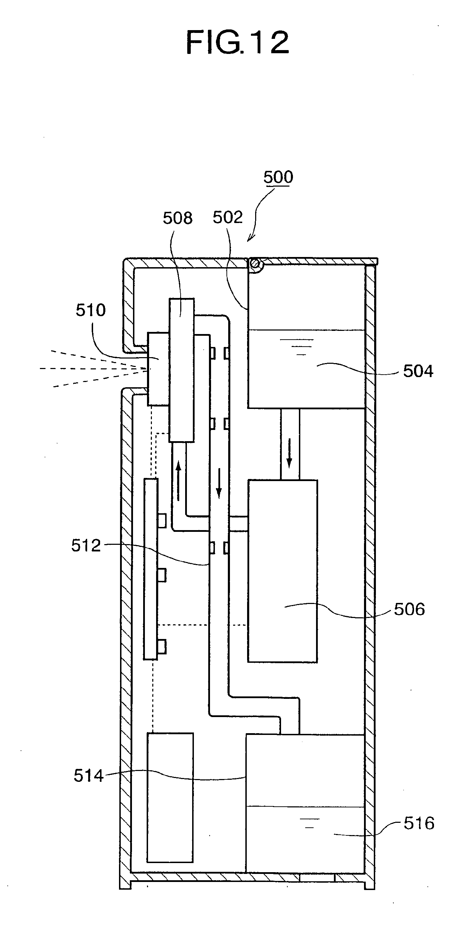

Fig. 12 is a schematic explanatory diagram showing an example of a conventional electrolytic water atomizer.

### FIRST BEST MODE FOR CARRYING OUT THE INVENTION

An example of an electrolytic water atomizer according to the present invention will be described below with reference to the drawings.

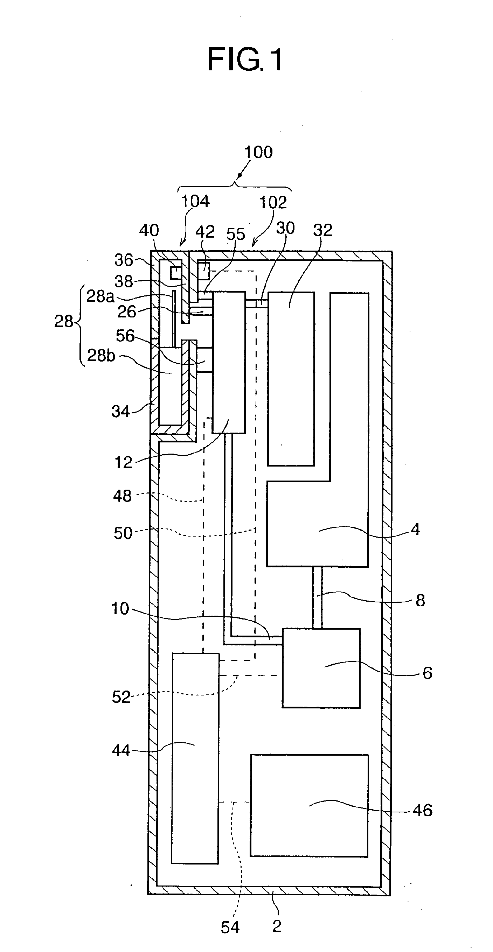

In Fig. 1, numeral 100 denotes an electrolytic water atomizer. The electrolytic water atomizer 100 comprises an electrolytic water atomizer body 102 and an atomizing unit 104 detachably attached to the electrolytic water atomizer body 102. Numeral 2 denotes a housing of the electrolytic water atomizer body 102 which is substantially a rectangular parallelepiped. Numeral 4 denotes an electrolytic aqueous solution tank housed within the housing 2. Electrolytic aqueous solution is contained in the electrolytic aqueous solution tank 4. Preferred examples of electrolytes are alkali metal salts and alkaline earth metal salts, such as sodium chloride, potassium chloride, and calcium chloride, as well as organic acids, e.g., ascorbic acid. An electrolyte concentration of 0.015 to 0.9 mass % is preferred.

A pump 6 is mounted below the electrolytic aqueous solution tank 4, and the electrolytic aqueous solution contained in the electrolytic aqueous solution tank 4 is supplied to the pump 6 through an electrolytic aqueous solution supply pipe 8. The electrolytic aqueous solution supplied to the pump 6 is then supplied under pressure to an electrolytic bath 12 through a delivery pipe 10 by means of the pump 6.

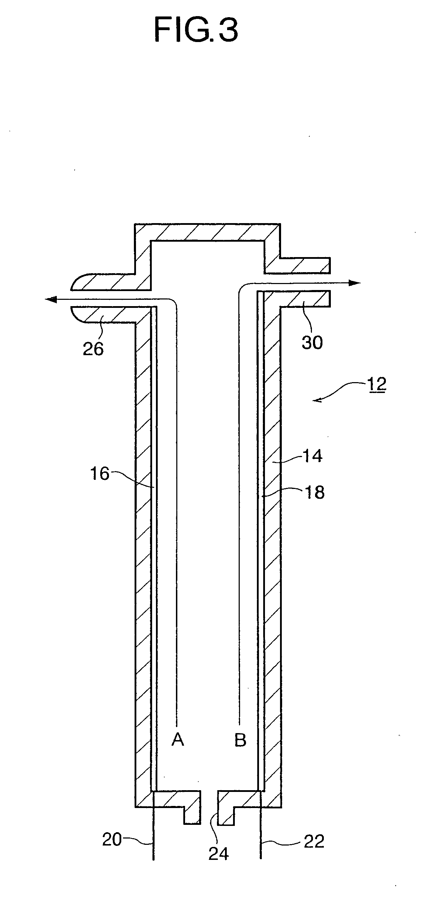

Fig. 3 is an enlarged diagram of the electrolytic bath 12. The electrolytic bath 12 is provided with an anode 16 and a cathode 18 which are disposed in parallel in a flat electrolytic bath housing 14. Numeral 20 denotes an anode terminal connected to the anode 16 and numeral 22 denotes a cathode terminal connected to the cathode 18. Both terminals 20 and 22 are drawn out of a lower end of the electrolytic bath housing 14 to the exterior.

The electrolytic aqueous solution supplied from the pump 6 is supplied into the electrolytic bath 12 through an inflow port 24 formed in a lower part of the electrolytic bath 12, then flows upward within the electrolytic bath 12 while maintaining the state of a laminar flow and is electrolyzed with a voltage applied between the anode 16 and the cathode 18. As a result, anode water (acidic water) is produced in the vicinity of the anode 16 and cathode water (alkaline water) is produced in the vicinity of the cathode 18.

Since the electrolytic aqueous solution present within the electrolytic bath 12 flows as a laminar flow, the produced anode water flows upward along a surface of the anode 16 as shown by an arrow A. Only the anode water passes through an electrolytic water outflow pipe 26 formed on the upper side of the electrolytic bath 12, and is supplied to an atomizing element 28 described later.

On the other hand, the cathode water produced within the electrolytic bath 12 flows upward along the cathode 18 as shown by an arrow B in Fig. 3, then passes through a discharge pipe 30 connected to the upper side of the electrolytic bath 12, then is fed to a waste liquid tank 32 described later, the waste liquid tank 32 being mounted above the pump 6, and is temporarily stored as waste liquid in the waste liquid tank 32. Within the housing 2, the waste liquid tank 32 is mounted above the pump 6 at approximately the same height as the electrolytic bath 12 and the electrolytic aqueous solution tank 4. The waste liquid tank 32 is positioned adjacent to the electrolytic aqueous solution tank 4.

In Fig. 1, in front of the outflow pipe 26 formed at the upper side of the electrolytic bath 12, the atomizing unit 104 is attached to the electrolytic water atomizer body 102. The atomizing unit 104 is adjacent to the electrolytic water aqueous solution tank 4 and the waste liquid tank 32, and openings of both tanks 4 and 32 are also adjacent thereto.

As an example of a method for mounting the atomizing unit 104, there is a conventional engaging means using a slide guide or a retaining projection etc.

As shown in Fig. 1, the atomizing unit 104 is made up of an atomizing unit body 34 which contains the atomizing element 28 and an atomizing unit opening/closing cover 36 attached to an upper part of the atomizing unit body 34.

The atomizing element 28 comprises a perforated plate 28a and a piezo-oscillator 28b. The perforated plate 28a is formed with a large number of through pores 18 to 24 µm in diameter and an end portion thereof is fixed to the piezo-oscillator 28b. When an alternating current or a pulse voltage is applied to the piezo-oscillator 28b, the piezo-oscillator 28b oscillates, with consequent oscillation of the perforated plate 28a fixed to the piezo-oscillator 28b. As a result, the electrolytic water supplied from the outflow pipe 26 is sprayed as fine droplets to the exterior through a large number of through pores formed in the perforated plate 28a as will be described later.

The atomizing unit opening/closing cover 36 is for exposing or hiding the perforated plate 28a of the atomizing element 28 and is attached to the atomizing unit body 34 so that it can slide vertically with respect to the atomizing unit body 34. A discharge port opening/closing means 38 constituted by a flat plate is suspended from an upper part of the cover 36 toward the outflow pipe 26 on the electrolytic water atomizer body 102 side of the atomizing unit opening/closing cover 36. When the cover 36 is closed so as to hide the perforated plate 28a, a discharge port at a front end of the outflow pipe 26 is closed with the discharge port opening/closing means 38, so that electrolytic water does not leak out from the discharge port of the outflow pipe 26.

A magnet 40 is attached to an upper part of the cover 36. The magnet 40 cooperates with a reed switch 42 attached to a corresponding part of the electrolytic water atomizer body 102 to detect a position of the cover 36.

Numeral 44 denotes a control unit with a microprocessor incorporated therein. The pump 6, the electrolytic bath 12, and the atomizing element 28 etc. are controlled by the control unit 44, and a signal from the reed switch 42 is inputted to the control unit 44. Numeral 46 denotes a power supply for the supply of electric power to the control unit 44, pump 6, electrolytic bath 12, and atomizing element 28 etc. Either a primary battery or a secondary battery is employable as the power supply 46.

Numeral 54 denotes a wiring line for supplying electric power from the power supply 46 to the control unit 44. Numeral 48 denotes a wiring line for supplying controlled electric power suitable for electrolysis to the electrolytic bath 12. Numeral 50 denotes a signal line connecting the reed switch 42 with the control unit 44. Numeral 52 denotes a wiring line for supplying controlled electric power from the control unit 44 to the pump 6.

Each of numerals 55 and 56 denotes a packing formed of an elastic material such as rubber and interposed between an inner wall of the housing 2 and the electrolytic bath 12. The packings 55 and 56 constitute a waterproof means. With the waterproof means, the entry of anode water into the electrolytic water atomizer body 102 is prevented.

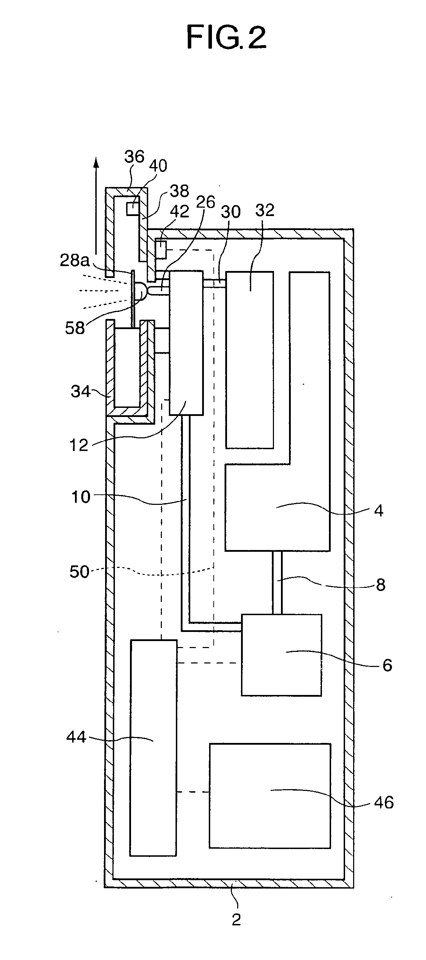

A description will now be given about spraying electrolytic water with use of the electrolytic water atomizer constructed as above. As shown in Fig. 2, first the cover 36 is pulled upward. As a result, the discharge port opening/closing means 38 also moves upward and the discharge port of the outflow pipe 26 becomes open from its closed state.

The magnet 40 attached to the cover 36 is also moved upward away from the reed switch 42. Upon sensing this state, the control unit 44 supplies electric power from the power supply 46 to the pump 6, the electrolytic bath 12, and the atomizing element 28, whereby the pump 6 operates and the electrolytic aqueous solution stored in the electrolytic aqueous solution tank 4 is fed to the electrolytic bath 12 through the electrolytic aqueous solution supply pipe 8, the pump 6, and the delivery pipe 10, and is electrolyzed therein.

The anode water produced on the anode side within the electrolytic bath 12 passes through the outflow pipe 26 and is supplied as droplets 58 from the discharge port formed at the front end of the outflow pipe 26 to the perforated plate 28a which is oscillating. Further, the anode water passes through the fine through pores formed on the perforated plate 28a and is sprayed to the front of the perforated plate 28a.

On the other hand, the cathode water passes through the discharge pipe 30 and is fed to the waste liquid tank 32, in which it is temporarily stored, then is discharged to the exterior as necessary.

For stopping the spray of electrolytic water at the end of completion of the spray, the cover 36 is pushed down. As a result, the magnet 40 approaches the reed switch 42 and a signal is sent to the control unit through the signal line 50. With this signal, the electric power supplied to the electrolytic bath 12 and the pump 6 is cut off and the spray of electrolytic water is stopped.

Although in the above example the reed switch 42 functions as a switch for electrolytic water spraying operation, no limitation is made thereto. There may be adopted a configuration wherein a power switch is provided separately and is turned ON after opening the cover 36 to start the spray of electrolytic water. In this case, if the spray of electrolytic water is started in accordance with AND condition of the reed switch 42 and the power switch, then even if the power switch is turned ON when the cover 36 is not pulled upward, the electrolytic water is not sprayed and thus an erroneous spray due to an erroneous operation can surely be prevented.

An electrolytic water spray defect may occur during long-term use of the electrolytic water atomizer according to the present invention. For example, the electrolytic water spray defect occurs due to the through pores of the perforated plate 28a being clogged by the electrolyte which results from drying and deposition of electrolytic water as adhered to the atomizing element. In the event of occurrence of the spray defect, the atomizing unit 104 is removed from the electrolytic water atomizer body 102 and replaced with a normal atomizing unit 104, whereby it is possible to remedy the spray defect.

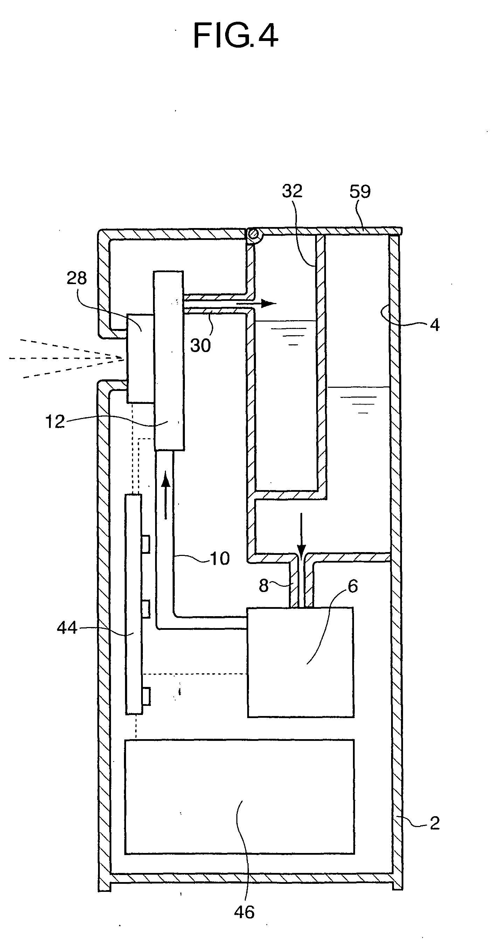

The shape and arrangement of the constituent parts of the electrolytic water atomizer according to the present invention are not limited to those shown in Fig. 1. For example, as shown in Fig. 4, the atomizing element 28 may be installed within the housing 2 and be fixed to the housing. Also in this case, as in Fig. 1, the electrolytic aqueous solution tank 4 and the waste liquid tank 32 are provided above the pump 6 and at approximately the same height as the electrolytic bath 12. By thus disposing both tanks 4 and 32 above the pump 6, it is possible to surely prevent the electrolytic aqueous solution from flowing from the electrolytic aqueous solution tank 4 to the waste liquid tank 32 due to a siphoning effect, even without providing any special mechanism. Besides, since any special mechanism is not provided, the number of part items and assembling man-hours are so much reduced and hence it is possible to attain the reduction of size and weight.

Further, since the opening of the aqueous solution tank 4 and the opening of the waste liquid tank 32 are disposed adjacent to each other above the pump, both tanks 4 and 32 can be closed with a single lid 59 in common. That is, it is possible to omit the lid of the waste liquid tank so far required in the conventional atomizer in which an aqueous solution tank and a waste liquid tank are separately provided above and below the pump. Accordingly, the configuration is simplified insofar as the lid is omitted and it is possible to attain the reduction of weight.

In Fig. 4, the other constructional points are almost the same as in Fig. 1, therefore, the same portions as in Fig. 1 are identified by the same reference numerals as in Fig. 1 and explanations thereof will here be omitted. In the present invention, modifications may be made within the scope not departing from the gist of the invention.

### SECOND BEST MODE FOR CARRYING OUT THE INVENTION

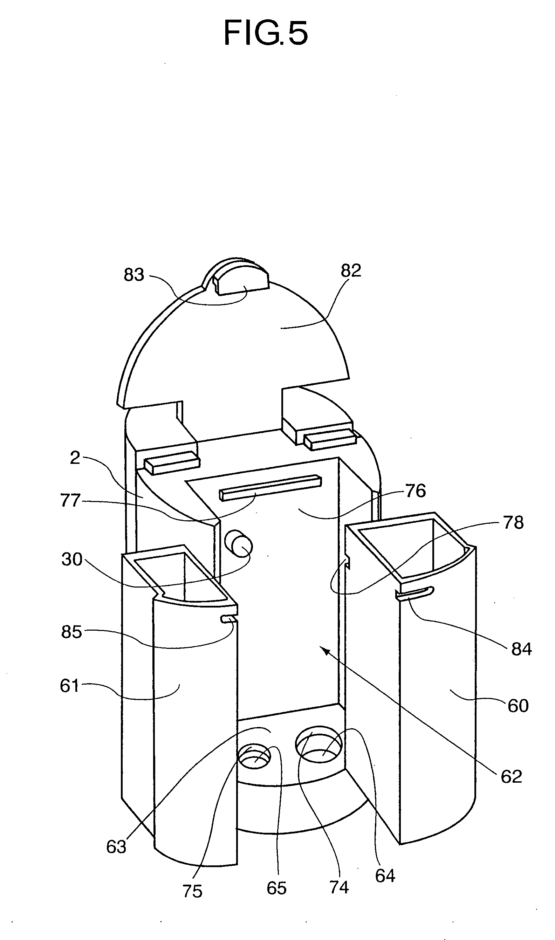

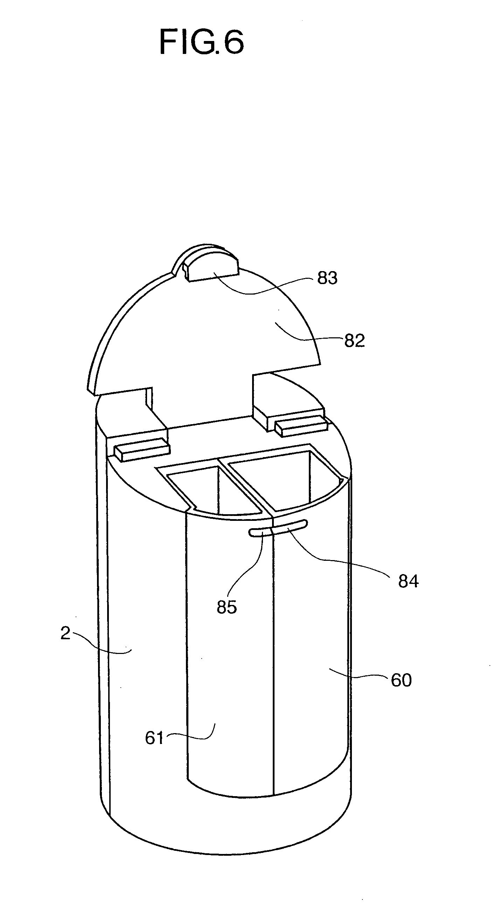

Next, another electrolytic water atomizer according to the present invention will be described with reference to Figs. 5 and 6. Fig. 5 shows an electrolytic aqueous solution tank and a waste liquid tank both used in the electrolytic water atomizer of the present invention and both in a removed state from the housing. Fig. 6 shows the electrolytic aqueous solution tank and the waste liquid tank both in a mounted state to the housing. In Figs. 5 and 6, the same reference numerals as in Fig. 1 denote the same members as in Fig. 1. In this example, an electrolytic aqueous solution tank 60 and a waste liquid tank 61 are formed separately from the housing 2 in such a manner that both tanks can be attached to and detached from the housing 2.

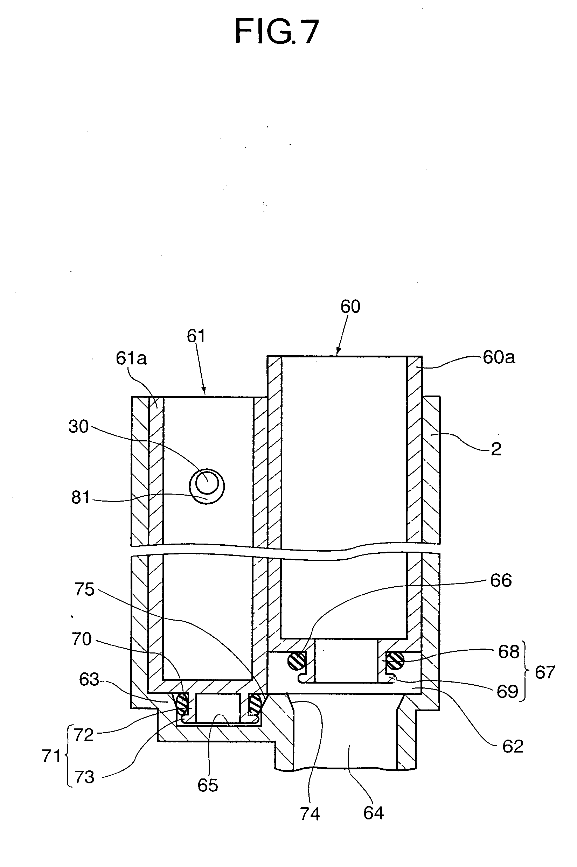

In the housing 2, as shown in Figs. 5 and 7, a space 62 is formed for receiving therein the electrolytic aqueous solution tank 60 and the waste liquid tank 61. The electrolytic aqueous solution tank 60 and the waste liquid tank 61 are mounted adjacent to each other within the space 62. Both tanks 60 and 61 are designed so that, when mounted to the housing 2, respective openings are adjacent to each other. Further, though not shown, both tanks 60 and 61 are designed so as to be positioned above the pump 6 as in Figs. 1 and 4.

In a bottom 63 of the housing 2 which forms a wall surface of the space 62 there is formed a through hole 64 as an elastic member receiving space in a position corresponding to the electrolytic aqueous solution tank 60. Likewise, in the bottom 63 of the housing 2 is formed a recess portion 65 as an elastic member receiving space in a position corresponding to the waste liquid tank 61. Although the elastic member receiving space in the bottom 63 corresponding to the electrolytic aqueous solution tank 60 is formed as the through hole 64 and the elastic member receiving space in the bottom 63 corresponding to the waste liquid tank 61 is formed as the recess portion 65, the elastic member receiving spaces are not limited to those shapes.

As shown in Fig. 7, a holding means 67 for holding an O-ring 66 as both an elastic member and a sealing member is formed integrally with a lower portion of a body 60a of the electrolytic aqueous solution tank 60. The holding member 67 comprises a cylindrical portion 68 for fitting the O-ring 66 on its outer periphery surface and a collar portion 69 formed at a lower end in the figure of the cylindrical portion 68 to prevent dislodgment of the O-ring 66. As in the electrolytic aqueous solution tank 60, also at a lower portion of a body 61a of the waste liquid tank 61, a holding member 71 for holding an O-ring 70 as an elastic member is formed integrally. The holding member 71 comprises a cylindrical portion 72 for fitting the O-ring 70 on its outer periphery surface and a collar portion 73 formed at a lower end in the figure of the cylindrical portion 72 to prevent dislodgment of the O-ring 70.

As shown in Figs. 5 and 7, in the vicinity of an inlet close to the space 62, an annular tapered wall surface 74 as an elastic member compressing wall surface is formed on an inner wall surface of the through hole 64 which is formed in the bottom 63. The tapered wall surface 74 is formed so that its inside diameter on the space 62 side is relatively large and becomes gradually smaller relatively with separating from the space 62. The size of the O-ring 66 and that of the tapered wall surface 74 are designed so that when the 0-ring 66 attached to the electrolytic aqueous solution tank 60 is inserted into the through hole 64, the O-ring 66 comes into contact with the tapered wall surface 74 of the through hole 64, and so that as the O-ring 66 is further inserted, the O-ring is compressed and its outside diameter contracts. In Fig. 7, the outside diameter of the O-ring 66 attached to the electrolytic aqueous solution tank 60 is shown in a state before the compression.

In the vicinity of an inlet close to the space 62, an annular tapered wall surface 75 as an elastic member compressing wall surface is formed in an inner wall surface of the recess portion 65 formed in the bottom 63. The tapered wall surface 75 is formed so that its inside diameter on the space 62 side is relatively large and gradually becomes smaller relatively with separating from the space 62. The size of the O-ring 70 and that of the tapered wall surface 75 are designed so that when the O-ring 70 attached to the waste liquid tank 61 is inserted into the recess portion 65, the O-ring 70 comes into contact with the tapered wall surface 75 of the recess portion 65, and so that as the O-ring 70 is further inserted, the O-ring 70 is compressed and its outside diameter contracts. In Fig. 7, the outside diameter of the O-ring 70 attached to the waste liquid tank 61 is shown in a state after the compression.

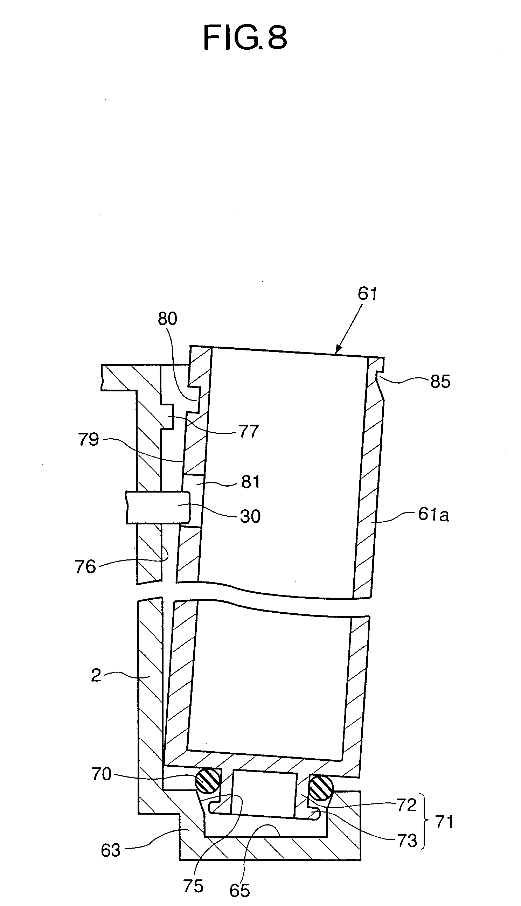

As shown in Figs. 5 and 8, a horizontally long engaging beam 77 as a first engaging means is formed at an upper part of a vertical wall surface 76 which forms the space 62 in the housing 2. As shown in Fig. 5, an engaging groove 78 as a second engaging means for engagement with the engaging beam 77 is formed in a wall surface of the electrolytic aqueous solution tank 60 opposed to the wall surface 76. Further, as shown in Fig. 8, an engaging groove 80 as a second engaging means for engagement with the engaging beam 77 is also formed in a wall surface 79 of the waste liquid tank 61 opposed to the wall surface 76. That is, the engaging groove 78 in the electrolytic aqueous solution tank 60 and the engaging groove 80 in the waste liquid tank 61 are formed so as to be engageable with the engaging beam 77 formed on the wall surface 76.

As shown in Fig. 8, the discharge pipe 30 is disposed so that a front end thereof projects from the wall surface 76 toward the space 62 side at a position somewhat lower than the engaging beam 77 on the wall surface 76. A through hole 81 for insertion therethrough of the front end of the discharge pipe 30 is formed in a position somewhat lower than the position where the engaging groove 80 in the waste liquid tank 61 is formed.

As shown in Fig. 5, a lid 82 for simultaneously opening and closing the upper openings of both electrolytic aqueous solution tank 60 and waste liquid tank 61 is attached pivotably to an upper end of the housing 2. A retaining projection 83 as a first retaining means is formed integrally at a free end of the lid 82. In the electrolytic aqueous solution tank 60, a retaining groove 84 as a second retaining means for engagement with the retaining projection 83 is formed on an outer surface portion on the side opposite to the side where the engaging groove 78 is formed. In the waste liquid tank 61, a retaining groove 85 as a second retaining means for engagement with the retaining projection 83 is formed on an outer surface on the side opposite to the side where the engaging groove 80 is formed. That is, the retaining projection 83 of the lid 82 is adapted to simultaneously engage both the retaining groove 84 formed in the electrolytic aqueous solution tank 60 and the retaining groove 85 formed in the waste liquid tank 61.

Next, reference will be made to the waste liquid tank 61 as an example and the following description is provided about mounting and removal of the waste liquid tank 61 to and from the housing 2. As shown in Fig. 8, the waste liquid tank 61 is inclined relative to the wall surface 76 of the housing 2 and a lower portion (the O-ring 70 and the holding member 71) of the waste liquid tank 61 is inserted into the recess portion 65 of the bottom 63. In the state shown in Fig. 8, the engaging beam 77 of the housing 2 is positioned higher than the engaging groove 80 of the waste liquid tank 61 and therefore, even if the waste liquid tank 61 is pushed in the horizontal direction, the engaging groove 80 of the waste liquid tank 61 does not come into engagement with the engaging beam 77 of the housing 2.

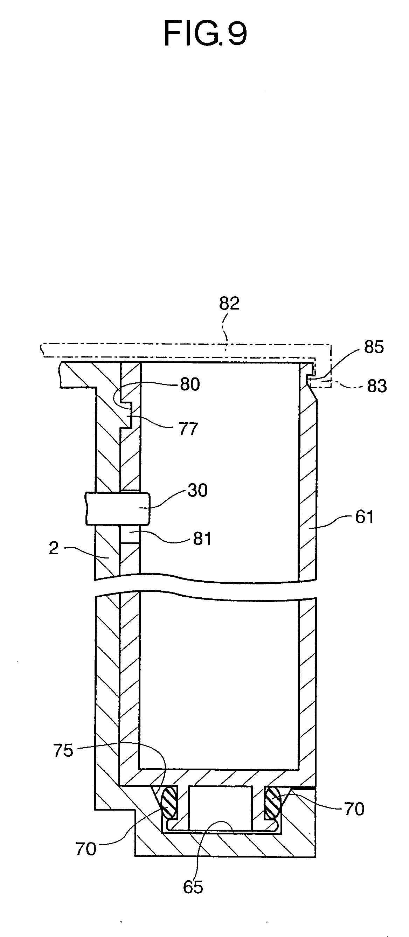

However, if the waste liquid tank 61 is pushed downward from the state of Fig. 8 in which the lower portion of the waste liquid tank 61 is fitted in the recess portion 65 of the bottom 63, the O-ring 70 is compressed and deformed by the tapered wall surface 75 and is received into the recess portion 65. As a result, the height of the engaging groove 80 of the waste liquid tank 61 shifts to the same height as the engaging beam 77 of the housing 2. Thereafter, if the upper portion of the waste liquid tank 61 is pushed in the horizontal direction, the engaging groove 80 of the waste liquid tank 61 comes into engagement with the engaging beam 77 of the housing 2 (Fig. 9). With the O-ring 70 received in the recess portion 65, at least either the outer periphery surface of the O-ring 70 is compressed by the tapered wall surface 75 or upper and lower surfaces of the O-ring 70 are sandwiched and compressed in between the bottom of the recess portion 65 and the body 61a of the waste liquid tank 61. As a result of the O-ring 70 being compressed, a restoring repulsive force acts on the O-ring 70, and with this repulsive force, force for moving the waste liquid tank 61 upward is applied to the same tank. With this upward force applied to the waste liquid tank 61, an urging force is exerted on a contact portion between the engaging groove 80 of the waste liquid tank 61 and the engaging beam 77 of the housing 2, so that the waste liquid tank 61 is no longer easily disengaged from the housing 2.

Next, for removing the waste liquid tank 61 from the housing 2, the tank 61 is once pushed downward to release the urging force of the O-ring 70 against the contact portion between the engaging groove 80 of the waste liquid tank 61 and the engaging beam 77 of the housing 2. Thereafter, by pulling the upper portion of the waste liquid tank 61 to this side horizontally, it is possible to remove the tank 61 from the housing 2.

The method for mounting and removal of the electrolytic aqueous solution tank 60 to and from the housing 2 is the same as the method for mounting and removal of the waste liquid tank 61 to and from the housing 2 described above. An electrolytic aqueous solution supplied into the electrolytic aqueous solution tank 60 is introduced into the pump 6 positioned below the same tank 60. Therefore, the O-ring 66 attached to the tank 60 functions not only as an elastic member but also as a sealing member for the housing 2.

In the state of Fig. 6 in which the electrolytic aqueous solution tank 60 and the waste liquid tank 61 are attached to the housing 2, as shown in Fig. 9, the openings of both the same tanks 60 and 61 are closed with the lid 82 and the retaining projection 83 formed at the front end of the lid 82 is engaged with the retaining grooves 84 and 85 of the tanks 60 and 61, respectively. As a result, the lid 82 never opens unless an external force is applied to the lid 82. Besides, unless the lid 82 opens, there is no fear of the electrolytic aqueous solution tank 60 and the waste liquid tank 61 being disengaged from the housing 2.

For taking out the electrolytic aqueous solution tank 60 and the waste liquid tank 61 from their closed state with the lid 82, first the retaining projection 83 on the lid 82 is disengaged from the retaining grooves 84 and 85 of both tanks 60 and 61 and the lid 82 is opened, then both tanks 60 and 61 are taken out from the housing 2.

### THIRD BEST MODE FOR CARRYING OUT THE INVENTION

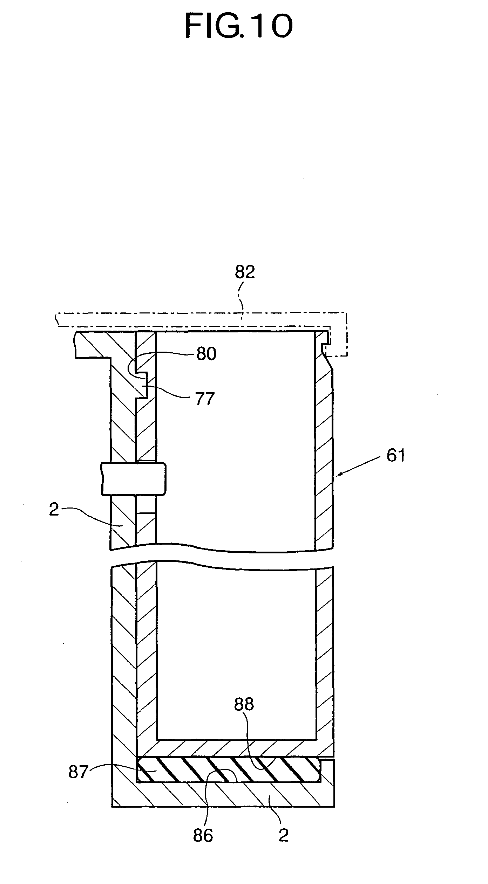

Next, another example in a state in which the waste liquid tank is attached to the housing will be described with reference to Fig. 10. In Fig. 10, the same reference numerals as in Figs. 7 and 8 represent the same members as in Figs. 7 and 8. A recess portion 86 is formed in the housing 2 at the position where the waste liquid tank 61 is mounted, and an elastic member 87 such as rubber is fitted in the recess portion 86 without dislodgment. The elastic member 87 is not limited to rubber. A bottom 88 of the waste liquid tank 61 serves as a wall surface which comes into contact with the elastic member 87. When the waste liquid tank 61 is attached to the housing 2, the bottom 88 of the tank 61 compresses the elastic member 87, causing deformation of the elastic member 87, and the repulsive force based on the compression of the elastic member 87 urges the waste liquid tank 61 upward in Fig. 10. With this upward urging force imposed on the waste liquid tank 61, the urging force is exerted on a contact portion between the engaging groove 80 of the tank 61 and the engaging beam 77 of the housing 2, with the result that the tank 61 becomes no longer easily disengageable from the housing 2.

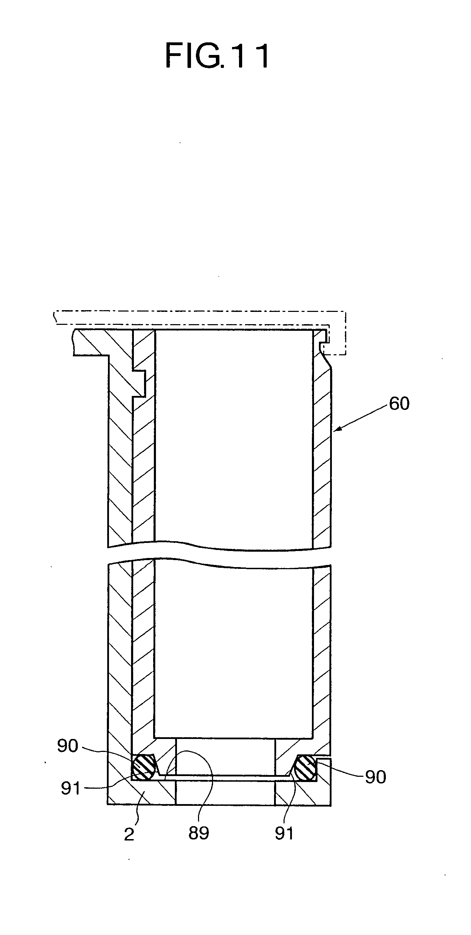

Fig. 11 shows another example of a mounted state of the electrolytic aqueous solution tank to the housing. In Fig. 11, the same reference numerals as in Figs. 7 and 5 represent the same members as in Figs. 7 and 5. A stepped portion 89 is formed in the housing 2 at the position where the electrolytic aqueous solution tank 60 is mounted, and an elastic member 90 such as an O-ring is fitted in the stepped portion 89 without dislodgment. The elastic member 90 is not limited to the O-ring. The lower portion of the electrolytic aqueous solution tank 60 is formed with a tapered wall surface 91 which comes into contact with the elastic member 90. When the electrolytic aqueous solution tank 60 is attached to the housing 2, the tapered wall surface 91 of the tank 60 compresses the elastic member 90, causing the elastic member 90 to be deformed, and a repulsive force based on the compression of the elastic member 90 urges the tank 60 upward in Fig. 11. With this upward urging force imposed on the electrolytic aqueous solution tank 60, an urging force is exerted on a contact portion between the engaging groove 78 of the tank 60 and the engaging beam 77 of the housing 2, so that the tank 60 becomes no longer easily disengageable from the housing 2.

### INDUSTRIAL APPLICABILITY

In the electrolytic water atomizer of the present invention, the electrolytic aqueous solution tank and the waste liquid tank are mounted above the pump. Consequently, a head difference between the electrolytic aqueous solution and the waste liquid becomes smaller, and without the provision of such a special mechanism as in the prior art, the electrolytic aqueous solution is surely prevented from flowing toward the waste liquid tank side under a siphoning effect. Besides, since any special mechanism is not provided, the number of part items and assembling man-hours are so much decreased and the reduction of both size and weight can be attained. Further, since both tanks are positioned near the atomizing unit, the distance required for laying the delivery pipe and the outflow pipe becomes shorter and hence it is possible to attain the reduction of both size and weight. As the pump, moreover, if there is used a pump of a small driving electric power, the electrolytic water atomizer becomes extremely advantageous as a portable atomizer driven by a battery.

Since the pump is positioned below both tanks, bubbles are difficult to enter the pump. So there is no fear of the pump racing due to the inclusion of bubbles therein and becoming incapable of supplying liquid. In the case where both tanks are positioned adjacent to each other above the pump, both tanks can be closed with a single lid although separate lids have heretofore been used to close the tanks. According to this configuration, the number of part items decreases, the reduction of weight and that of the number of processes can be attained, the replenishment of electrolytic aqueous solution and the discharge of waste liquid become easier, and the amount of the electrolytic aqueous solution and that of the waste liquid contained in both tanks can be easily checked visually. Further, in the case where the electrolytic aqueous solution tank and the waste liquid tank are constructed removably, the replenishment of electrolytic aqueous solution and the discharge of waste liquid become much easier.

According to the present invention there may be adopted a configuration wherein the electrolytic aqueous solution tank and the waste liquid tank are formed separately from the housing so that they can be attached to and detached from the housing. When the waste liquid tank is to be removed from the housing, it is tilted. By tilting the waste liquid tank, at the time of removing the waste liquid tank from the housing, the waste liquid contained in the waste liquid tank can be prevented from splashing up and spilling from the tank. Besides, when the electrolytic aqueous solution tank and the waste liquid tank are attached to the housing, the elastic member for urging both tanks in the predetermined direction is compressed, and with a repulsive force based on the compression, the engaged and fixed state of both tanks to the housing can be stabilized and it is possible to prevent both tanks from being easily disengaged from the housing. Further, by providing a first retaining means in the lid and a second retaining means for engagement with the first retaining means of the lid in each of the electrolytic aqueous solution tank and the waste liquid tank, the lid is locked to both tanks when the openings of both tanks are closed with the lid, so that the closure of the openings of both tanks is ensured and disengagement of both tanks from the housing can be prevented more positively.

The reason why the waste liquid tank is made removable from the housing is that the waste liquid staying in the waste liquid tank can be easily discharged and that the waste liquid tank can be taken out and washed. Likewise, the reason why the electrolytic aqueous solution tank is made removable from the housing is that the same tank can be easily washed. Making the electrolytic aqueous solution tank removable from the housing is advantageous in that the opening of the electrolytic aqueous solution tank is relatively small, so at the time of supplying an electrolytic aqueous solution to the same tank from a dedicated liquid tank, the electrolytic aqueous solution tank can be displaced into an inclined state and hence it becomes easier to pour the electrolytic aqueous solution.

## Claims

1. An electrolytic water atomizer comprising:
a housing;
an electrolytic aqueous solution tank provided within said housing;
a pump for supplying an electrolytic aqueous solution stored in said electrolytic aqueous solution tank to the inlet side of an electrolytic bath;
said electrolytic bath which is a flow type and is provided with an anode and a cathode opposed to each other in the interior thereof;
an atomizing unit for spraying electrolytic water produced on one electrode side in said electrolytic bath to the exterior of said housing;
a waste liquid tank;
a discharge pipe which connects said electrolytic bath with said waste liquid tank to conduct electrolytic water produced on the other electrode side in said electrolytic bath to said waste liquid tank;
a control unit for controlling the operation of said atomizing unit, said electrolytic bath, and said pump; and
a power supply for driving said components,
wherein said electrolytic aqueous solution tank and said waste liquid tank are disposed above said pump.

2. An electrolytic water atomizer according to claim 1, wherein said waste liquid tank is formed separately from said housing so that it can be attached to and detached from said housing, an elastic member is attached to one of said waste liquid tank and said housing, the other of said waste liquid tank and said housing has a wall surface adapted to contact with and compress said elastic member, a first engaging means is provided in said waste liquid tank, a second engaging means for engagement with said first engaging means is provided in said housing, and said first engaging means and said second engaging means are kept engaged with each other with the repulsive force of said elastic member compressed by said wall surface.

3. An electrolytic water atomizer according to claim 2, wherein said elastic member is an O-ring attached to a lower portion of said waste liquid tank, and said wall surface is an annular tapered wall surface formed in said housing.

4. An electrolytic water atomizer according to claim 2, wherein a lid is provided in said housing, a first retaining means is provided in said lid, and a second retaining means for engagement with said first retaining means is provided in said waste liquid tank.

5. An electrolytic water atomizer according to claim 2, wherein said electrolytic aqueous solution tank is formed separately from said housing so that it can be attached to and detached from said housing, an elastic member is attached to one of said electrolytic aqueous solution tank and said housing, the other of said electrolytic aqueous solution tank and said housing has a wall surface adapted to contact with and compress said elastic member, a first engaging means is provided in said electrolytic aqueous solution tank, a second engaging means for engagement with said first engaging means is provided in said housing, and said first engaging means and said second engaging means are kept engaged with each other with the repulsive force of said elastic member compressed by said wall surface.

6. An electrolytic water atomizer according to claim 5, wherein said elastic member is an O-ring attached to a lower portion of said electrolytic aqueous solution tank, and said wall surface is an annular tapered wall surface formed in said housing.

7. An electrolytic water atomizer according to claim 5, wherein a lid is provided in said housing, a first retaining means is provided in said lid, and a second retaining means for engagement with said first retaining means is provided in said waste liquid tank.
